# EUROPEAN PATENT APPLICATION

(11) **EP 4 810 013 A1**
(43) Date of publication of application: **23.09.2026**
(21) Application number: 24891642.1
(22) Date of filing: 28.09.2024
(51) Int. Cl.: A61K 8/9789, A61K 8/9771, A61Q 19/00, A61Q 19/08, C12N 5/04

(54) **COSMETIC COMPOSITION CONTAINING EXOSOMES DERIVED FROM ULLEUNG CHRYSANTHEMUM AND GINKGO LEAF AS ACTIVE INGREDIENT**

(30) Priority: 14.11.2023 KR 20230157326
(71) Applicant: Abio Materials Co., Ltd., Suwon-si, Gyeonggi-do 16229 (KR)
(72) Inventor: YOON, Eun Jeong, Suwon-si Gyeonggi-do 16398 (KR); KIM, Min Ha, Seongnam-si Gyeonggi-do 13232 (KR); KIM, Yun Jeong, Gwangju-si Gyeonggi-do 12777 (KR); PARK, Si Jun, Suwon-si Gyeonggi-do 16223 (KR); LEE, Hyun Sang, Anyang-si Gyeonggi-do 13972 (KR); KIM, Jung Soo, Anseong-si Gyeonggi-do 17550 (KR)
(74) Representative: Isarpatent
(86) International application number: PCT/KR2024/014815
(87) International publication number: WO 2025/105695

(57) **Abstract**

The present invention relates to a cosmetic composition containing Ulleung chrysanthemum exosomes and Ginkgo biloba leaf exosomes as active ingredients, and more particularly, to a cosmetic composition containing, as an active ingredient, Ulleung chrysanthemum exosomes and/or Ginkgo biloba leaf exosomes purified using a freeze-thaw treatment, a UV pretreatment, and an aqueous two-phase system, the cosmetic composition having excellent effects of wrinkle improvement, cell regeneration, and skin barrier enhancement.

## Description

### BACKGROUND

### Technical Field

The present invention relates to a cosmetic composition containing exosomes derived from Ulleung chrysanthemum and Ginkgo biloba leaf as an active ingredient, and more particularly, to a cosmetic composition containing, as an active ingredient, Ulleung chrysanthemum exosomes and/or Ginkgo biloba leaf exosomes purified using a freeze-thaw treatment, a UV pretreatment, and an aqueous two-phase system, the cosmetic composition having excellent effects of wrinkle improvement, cell regeneration, and skin barrier enhancement.

The present invention is a result of research conducted as part of the Science Belt Support Project supported by the Ministry of Science and ICT and the Korea Innovation Foundation. (Project No.: 2023-SB-SB-0010-01)

### Background Art

The functions of cosmetics have expanded beyond cleansing and simple makeup to removing or inhibiting causes of skin aging, such as pollutants, stress, ultraviolet rays, reactive oxygen species, and peroxides. As society has entered an aging society worldwide, the market for cosmetics for preventing skin aging has continuously increased, and having clean and healthy skin has become a matter of great interest to modern women. Accordingly, demand for raw materials having wrinkle improvement effects and cosmetics using the same has continuously increased. In addition, skin aging is closely related to moisture supply and moisture retention. Skin dryness results from a decrease in cohesion of keratinocytes caused by moisture loss, that is, abnormal function of the skin barrier.

Recently, cosmetic compositions having excellent wrinkle improvement and skin barrier enhancement effects using safe natural materials have been actively developed. The present inventors have completed the present invention by studying raw materials that do not cause skin irritation and have effects such as wrinkle improvement, cell regeneration, and skin barrier enhancement.

As used herein, the term "exosome" refers to a small membrane-structured vesicle secreted from various cells, and is defined as a type of extracellular vesicle (EV). All cells exchange information with other cells or external environments, and secrete extracellular vesicles for such exchange. Exosomes have a size of about 50 nm to 200 nm and include physiologically active substances, such as proteins, lipids, and nucleic acids. Exosomes are present in various cells, such as mammals, bacteria, and plants, and reflect the state of the cells from which the exosomes originate, and thus may be used for diagnosis and treatment. Exosomes are phospholipid bilayer structures, readily penetrate into cells, and perform various physiological and pathological functions, such as immune responses and signal transduction.

Recently, studies have been conducted on various efficacies of exosomes derived from plants. Plant-derived exosomes are natural nanoparticles that contain physiologically active substances and signaling substances secreted by plant cells themselves and assist in intercellular movement and absorption. Exosomes purified from plants are known to have no toxicity compared with mammal-derived exosomes.

Such exosomes are materials that can be used in fields such as pharmaceuticals, cosmetics, and foods due to various advantages and activities thereof. However, due to structural characteristics of being composed of a phospholipid bilayer, the exosomes have low dispersibility and a tendency to aggregate. In addition, the exosomes are unstable at high temperatures and may be easily broken during a process of preparing a cosmetic formulation, and these properties may reduce the stability of the exosomes in the formulation and cause precipitation. Therefore, in order to continuously maintain activity, there is a need to increase the solubility and dispersibility of exosomes in an aqueous solution and thereby improve stability in a formulation.

The present inventors conducted intensive studies to prepare plant-derived exosomes exhibiting excellent skin condition-improving activity by applying various methods and to use the plant-derived exosomes in a cosmetic composition. As a result, the present inventors confirmed that Ulleung chrysanthemum exosomes and/or Ginkgo biloba leaf exosomes, which are purified using a freeze-thaw treatment and a UV pretreatment under specific conditions and an aqueous two-phase system, exhibit excellent skin-improving efficacy, thereby completing the present invention.

### SUMMARY

### Technical Problem

An object of the present invention is to provide a cosmetic composition containing, as an active ingredient, Ulleung chrysanthemum exosomes, Ginkgo biloba leaf exosomes, or a mixture thereof, the cosmetic composition having excellent stability and excellent effects of wrinkle improvement, cell regeneration, and skin barrier enhancement.

Another object of the present invention is to provide a method for purifying Ulleung chrysanthemum exosomes or Ginkgo biloba leaf exosomes.

### Technical Solution

To achieve the above object, according to the present invention, there is provided a cosmetic composition containing, as an active ingredient, Ulleung chrysanthemum exosomes, Ginkgo biloba leaf exosomes, or a mixture thereof.

The mixed exosomes are formed by mixing Ulleung chrysanthemum exosomes and Ginkgo biloba leaf exosomes at a weight ratio of 1 to 3:1 to 3.

The Ulleung chrysanthemum exosomes and the Ginkgo biloba leaf exosomes as the active ingredient are purified by a method including:
(A) adding a solvent to Ulleung chrysanthemum or Ginkgo biloba leaves and extracting by repeating freezing and thawing;
(B) treating Ulleung chrysanthemum or Ginkgo biloba leaves subjected to freeze-thaw pretreatment with UV-A for 1 hour to 6 hours;
(C) pressing the UV-treated Ulleung chrysanthemum or Ginkgo biloba leaves to obtain a pressed juice;
(D) centrifuging the pressed juice at 1,000×g to 10,000×g to obtain a supernatant;
(E) lyophilizing the supernatant in which exosomes are present;
(F) forming an aqueous two-phase system using polyethylene glycol (PEG)/dextran in the lyophilized product; and
(G) obtaining a lower phase in which exosomes are concentrated from the aqueous two-phase system.

More preferably, the freeze-thaw pretreatment in step (A) includes adding water to Ulleung chrysanthemum or Ginkgo biloba leaves and repeating, 2 to 5 times, a process of freezing at a temperature of -80°C to -70°C for 15 hours to 20 hours and then thawing at a temperature of 40°C to 50°C for 8 hours to 10 hours. Preferably, the UV treatment in step (B) is performed with UV-A having a wavelength of 340 nm to 380 nm for 4 hours to 6 hours, and more preferably, with UV-A having a wavelength of 365 nm for 6 hours.

The Ulleung chrysanthemum exosomes, the Ginkgo biloba leaf exosomes, or the mixture thereof as the active ingredient are contained in an amount of 0.0001% to 30.0% (w/w) based on a total weight of the composition.

The cosmetic composition is characterized by being for wrinkle improvement, cell regeneration, or skin barrier enhancement.

To achieve the other object, according to the present invention, there is provided a method for purifying Ulleung chrysanthemum or Ginkgo biloba leaf exosomes, the method including:
(A) adding a solvent to Ulleung chrysanthemum or Ginkgo biloba leaves and extracting by repeating freezing and thawing;
(B) treating Ulleung chrysanthemum or Ginkgo biloba leaves subjected to freeze-thaw pretreatment with UV-A for 1 hour to 6 hours;
(C) pressing the UV-treated Ulleung chrysanthemum or Ginkgo biloba leaves to obtain a pressed juice;
(D) centrifuging the pressed juice at 1,000×g to 10,000×g to obtain a supernatant;
(E) lyophilizing the supernatant in which exosomes are present;
(F) forming an aqueous two-phase system using polyethylene glycol (PEG)/dextran in the lyophilized product; and
(G) obtaining a lower phase in which exosomes are concentrated from the aqueous two-phase system.

More preferably, the freeze-thaw treatment in step (A) is performed by repeating, 2 to 5 times, a process of freezing at a temperature of -80°C to -70°C for 15 hours to 20 hours and then thawing at a temperature of 40°C to 50°C for 8 hours to 10 hours, and the UV treatment in step (B) is performed with UV-A having a wavelength of 340 nm to 380 nm for 4 hours to 6 hours, and more preferably, with UV-A having a wavelength of 365 nm for 6 hours.

### Effects of the Invention

The Ulleung chrysanthemum exosomes, Ginkgo biloba leaf exosomes, or a mixture thereof of the present invention, which are purified using a freeze-thaw treatment, a UV pretreatment, and an aqueous two-phase system, have excellent stability and exhibit excellent wrinkle improvement effects, cell regeneration effects, and skin barrier enhancement effects, and thus can be usefully used as a cosmetic material for skin improvement.

### BRIEF DESCRIPTION OF THE DRAWINGS

FIG. 1 is a TEM image of exosomes derived from Ulleung chrysanthemum and purified according to an embodiment of the present invention.
FIG. 2 is a TEM image of exosomes derived from Ginkgo biloba leaf and purified according to an embodiment of the present invention.
FIG. 3 is a graph showing NTA results for confirming a size distribution and a particle number of exosome particles derived from Ulleung chrysanthemum and purified according to an embodiment of the present invention.
FIG. 4 is a graph showing NTA results for confirming a size distribution and a particle number of exosome particles derived from Ginkgo biloba leaf and purified according to an embodiment of the present invention.
FIG. 5 is a graph showing MTT assay results for cytotoxicity of exosomes derived from Ulleung chrysanthemum and Ginkgo biloba leaf and purified according to the present invention.
FIG. 6 is a graph showing results of evaluating wrinkle improvement efficacy of exosomes derived from Ulleung chrysanthemum and Ginkgo biloba leaf and purified according to the present invention based on COL1A1 expression.
FIG. 7 is a graph showing results of evaluating wrinkle improvement efficacy of exosomes derived from Ulleung chrysanthemum and Ginkgo biloba leaf and purified according to the present invention based on inhibition of MMP-1 expression.
FIG. 8 is a graph showing results of evaluating skin barrier enhancement efficacy of exosomes derived from Ulleung chrysanthemum and Ginkgo biloba leaf and purified according to the present invention based on Filaggrin expression.
FIG. 9 is a graph showing results of evaluating skin barrier enhancement efficacy of exosomes derived from Ulleung chrysanthemum and Ginkgo biloba leaf and purified according to the present invention based on Loricrin expression.
FIG. 10 is an image showing results of evaluating cell regeneration efficacy of exosomes derived from Ulleung chrysanthemum and Ginkgo biloba leaf and purified according to the present invention by a wound healing assay.
FIG. 11 is a graph showing results of evaluating cell regeneration efficacy of exosomes derived from Ulleung chrysanthemum and Ginkgo biloba leaf and purified according to the present invention based on a wound area ratio.

### DETAILED DESCRIPTION

Hereinafter, the present invention will be described in more detail.

Plant-derived exosomes contain physiologically active substances and signaling substances secreted by plant cells themselves, and are known to have no toxicity compared with mammal-derived exosomes. Due to these advantages, plant-derived exosomes are materials that can be used in fields such as pharmaceuticals, cosmetics, and foods. However, due to structural characteristics of being composed of a phospholipid bilayer, plant-derived exosomes have low dispersibility and a tendency to aggregate, and thus have a problem in that it is difficult to continuously maintain their activity in a formulation. The present invention is technically characterized by separating and purifying, with high purity, exosomes having excellent skin-improving activity from Ulleung chrysanthemum and Ginkgo biloba leaves through a freeze-thaw treatment and a UV pretreatment, and using the exosomes as a cosmetic material.

Ulleung chrysanthemum, commonly known as Dendranthema zawadskii var. lucidum (Nakai), grows in mountainous areas of Seonginbong Peak on Ulleungdo Island, Gyeongsangbuk-do. The leaves are deeply divided into relatively narrow lobes, and the lobes are lanceolate, thick, glossy, and petiolate. Ulleung chrysanthemum is similar in appearance to Gujeolcho plants of the Chrysanthemum zawadskii group, but is characterized in that the leaf lobes are more deeply divided. Ulleung chrysanthemum is one of the rare and endangered plant species designated by the Korea Forest Service. Ulleung chrysanthemum was first discovered by Nakai of Japan and reported to an academic society in Japan. Subsequent studies revealed that Ulleung chrysanthemum is an endemic plant found only on Ulleungdo Island in Korea. The natural habitat of Ulleung chrysanthemum has gradually disappeared due to indiscriminate collection, and an area where Ulleung chrysanthemum grows in Nari Basin has been designated and protected as a natural monument. Ulleung chrysanthemum has efficacies such as warming the middle, regulating menstruation, and promoting digestion, and has been used as a medicinal material for treating irregular menstruation, coldness of the uterus, infertility, stomach coldness, and dyspepsia. Ulleung chrysanthemum contains chlorogenic acid, luteoloside, cosmosiin, linarin, and the like, and has high total flavonoid and polyphenol contents, which may contribute to excellent antioxidant activity.

Unlike leaves of most gymnosperms, such as pine trees, juniper trees, spruce trees, and fir trees, which are needle-shaped, Ginkgo biloba leaves are fan-shaped and divided at a middle portion thereof. Ginkgo biloba leaves are alternately borne on long shoots, and three to five leaves are densely and alternately borne on short shoots so as to appear as if the leaves grow from one place. In addition, leaves on long shoots are deeply divided, whereas many leaves on short shoots have smooth margins. New leaves emerge in spring, turn yellow in autumn, and then fall. In addition to flavonoids, Ginkgo biloba leaves contain active components such as ginkgolides, bilobalides, and terpenoids, and have excellent antioxidant effects of removing reactive oxygen species harmful to the human body. In addition, Ginkgo biloba leaves are known to improve blood circulation by inhibiting platelet aggregation and inducing vasodilation through activation of nitric oxide (NO) and prostaglandin I2 (PGI2, prostacyclin). In addition, Ginkgo biloba leaves are also known to have a memory-enhancing effect and to be capable of improving cognitive ability in dementia.

Exosomes derived from Ulleung chrysanthemum and Ginkgo biloba leaf as active ingredients of the cosmetic composition of the present invention are purified by the following method.

The exosomes are purified by a method including: (A) adding a solvent to Ulleung chrysanthemum or Ginkgo biloba leaves and extracting by repeating freezing and thawing; (B) treating Ulleung chrysanthemum or Ginkgo biloba leaves subjected to the freeze-thaw pretreatment with UV-A for 1 hour to 6 hours; (C) pressing the UV-treated Ulleung chrysanthemum or Ginkgo biloba leaves to obtain a pressed juice; (D) centrifuging the pressed juice at 1,000×g to 10,000×g to obtain a supernatant; (E) lyophilizing the supernatant in which exosomes are present; (F) forming an aqueous two-phase system using polyethylene glycol (PEG)/dextran in the lyophilized product; and (G) obtaining a lower phase in which exosomes are concentrated from the aqueous two-phase system.

In the present invention, in order to prepare exosomes derived from Ulleung chrysanthemum and Ginkgo biloba leaf having excellent skin-improving activity, water is first added to raw materials, and a freeze-thaw treatment and a UV pretreatment are performed.

In plants, exosome secretion characteristics may vary depending on environmental factors. In particular, when plants are subjected to stress, the amount of exosome secretion may be affected. Accordingly, in order to identify optimal conditions under which plants secrete exosomes, yields and skin-improving activities were confirmed through various pretreatments. As a result, it was confirmed that, when Ulleung chrysanthemum and Ginkgo biloba leaves are subjected to a freeze-thaw treatment and a UV pretreatment as pretreatments, exosome secretion is increased and skin-improving activity is increased.

According to a preferred embodiment of the present invention, the freeze-thaw treatment in step (A) is performed by repeating, 2 to 5 times, a process of freezing at a temperature of -80°C to -70°C for 15 hours to 20 hours and then thawing at a temperature of 40°C to 50°C for 8 hours to 10 hours. Subsequently, UV-A irradiation is performed at a wavelength of 340 nm to 380 nm for 4 hours to 6 hours (step (B)).

The UV treatment was performed after confirming that, when plant exosomes were subjected to UV treatment, there was no change in the size and concentration of the exosomes.

In step (C), it is preferable that a screw used in the pressing process has a stirring speed of 20 rpm to 50 rpm.

The centrifugation used in step (D) refers to a method of separating particles in a solution using centrifugal force according to size, shape, density, viscosity, and rotor speed. It is necessary to remove large contaminants by sequentially adjusting rpm, and in order to obtain a final solution for forming an aqueous two-phase system, it is more preferable to perform centrifugation at 10,000×g.

The lyophilization used in step (E) is a method of drying a material by rapidly lowering the temperature of a container to freeze the material to be dried, and then making the pressure inside the container close to a vacuum, thereby directly sublimating a solidified solvent contained in the material into water vapor. Freezing is performed at -50°C to -80°C for 15 hours to 24 hours, and drying is performed in a lyophilizer under vacuum for 72 hours to 120 hours. In this case, the vacuum state generally refers to a pressure state of the lyophilizer.

In order to efficiently separate and purify exosomes, the present invention uses an aqueous two-phase partitioning method, which is a separation method that forms two phases using two types of aqueous solutions that are not readily miscible with each other and separates a material using a difference in affinity for each phase.

In general, PEG/salt, such as sulfate, phosphate, citrate, or the like, may also be used to form an aqueous two-phase system. However, in order to achieve the object of the present invention, it is preferable to use PEG/dextran. Dextran is a natural polymer obtained by bacterial action, and is used as a thickener, a binder, and a bulking agent in cosmetic formulations.

In the formation of the aqueous two-phase system in step (F), PEG having a molecular weight of 10,000 to 35,000 is used in an amount of 1% to 15% by weight, preferably 2% to 5% by weight, and dextran having a molecular weight of 300,000 to 650,000 is used in an amount of 1% to 8% by weight, preferably 1% to 3% by weight. When the PEG and dextran are used at a concentration ratio of 3.3% by weight:1.7% by weight, the yield of exosomes is the highest and the stability is the greatest, which is more preferable.

In order to increase the purity of exosomes, after step (G), an additional process may be performed in which forming an aqueous two-phase system using an aqueous two-phase system solution having the same concentration and obtaining a lower phase are repeated 2 to 3 times.

Ulleung chrysanthemum exosomes, Ginkgo biloba leaf exosomes, and mixed exosomes thereof prepared by the above method exhibited superior wrinkle improvement effects (Test Example 7 and Test Example 8), cell regeneration effects (Test Example 11), and skin barrier enhancement effects (Test Example 9 and Test Example 10), compared with ordinary Ulleung chrysanthemum extracts and Ginkgo biloba leaf extracts.

Therefore, the exosomes derived from Ulleung chrysanthemum and Ginkgo biloba leaf may be used in a cosmetic composition for wrinkle improvement, cell regeneration, and skin barrier enhancement. In this case, the exosomes derived from Ulleung chrysanthemum and Ginkgo biloba leaf as the active ingredient, or mixed exosomes thereof, may be contained in an amount of 0.0001% to 30.0% (w/w) based on the total weight of the cosmetic composition. The mixed exosomes are formed by mixing Ulleung chrysanthemum exosomes and Ginkgo biloba leaf exosomes at a weight ratio of 1 to 3:1 to 3.

The cosmetic composition may be prepared in any commonly manufactured formulation, and may be prepared, for example, as a skin lotion, a skin toner, a facial pack, a nourishing cream, a moisturizing cream, an essence, a body cream, a body lotion, a body oil, a cleansing foam, a cleansing lotion, a soap, a patch, a foundation, a lipstick, a makeup base, a lipstick, or the like.

Hereinafter, the present invention will be described in more detail with reference to the following Examples and Test Examples. However, the following Examples are provided only to illustrate the present invention, and the present invention is not limited by the following Examples. It will be apparent to those of ordinary skill in the art to which the present invention pertains that substitutions and equivalent other embodiments may be made without departing from the technical spirit of the present invention.

### Example 1: Preparation of Ulleung chrysanthemum Exosomes

### Freeze-Thaw Pretreatment of Ulleung chrysanthemum

Purified water was added to 100 g of Ulleung chrysanthemum, followed by freezing at a temperature of -80°C for 18 hours and thawing at a temperature of 40°C for 8 hours. This process was repeated three times in total, thereby placing the material in a state in which exosomes can be readily extracted.

### UV-A Treatment of Ulleung chrysanthemum

The freeze-thaw-pretreated Ulleung chrysanthemum was treated with UV-A having a wavelength of 365 nm for 6 hours.

### Pressing of Ulleung chrysanthemum

The UV-treated Ulleung chrysanthemum was pressed using a general juicer with a low-speed screw at 30 rpm, and the obtained Ulleung chrysanthemum pressed juice was filtered through a mesh screen to remove suspended matter. The recovered Ulleung chrysanthemum pressed juice was stored at -80°C before being subjected to purification.

### Recovery of Supernatant for Exosome Purification

Since large contaminants needed to be removed from the Ulleung chrysanthemum pressed juice for exosome purification, centrifugation was performed at 10,000×g for 10 minutes at 4°C. After centrifugation, the supernatant was recovered to form an aqueous two-phase system.

### Lyophilization of Supernatant

Lyophilization was performed for the purpose of reducing the volume of the supernatant for mass production of exosomes. Freezing was performed at -80°C for 20 hours, and drying was performed in a lyophilizer under vacuum for 100 hours. In this case, the vacuum state generally refers to a pressure state of the lyophilizer, and the freezing and drying times may vary depending on the volume of the solution.

### Formation of Aqueous Two-Phase System

Purified water was added to the lyophilized supernatant, and an aqueous two-phase system was formed using PEG/dextran. An aqueous two-phase system was formed using 3.3% by weight of PEG (purchased from Sigma-Aldrich) having a molecular weight of 10,000 to 35,000 and 1.7% by weight of dextran (purchased from Sigma-Aldrich) having a molecular weight of 300,000 to 650,000.

### Recovery of Ulleung chrysanthemum Exosomes

After the supernatant and the PEG/dextran solution were mixed, centrifugation was performed at 1,000×g for 10 minutes at 4°C. After centrifugation, the supernatant was removed to recover exosomes.

### Additional Washing Process

In order to increase purity, an additional washing process was performed by adding the aqueous two-phase system solution having the same concentration to the recovered lower phase. After three repeated treatments, a final lower phase in which exosomes were concentrated was recovered.

### Example 2: Preparation of Ginkgo biloba Leaf Exosomes

Exosomes derived from Ginkgo biloba leaf were purified in the same manner as in Example 1.

### Example 3: Preparation of Mixed Exosomes of Ulleung chrysanthemum and Ginkgo biloba Leaf

The Ulleung chrysanthemum exosomes and the Ginkgo biloba leaf exosomes prepared in Examples 1 and 2 were mixed at the same weight ratio to prepare mixed exosomes.

### Examples 4 to 9: Preparation of Ulleung chrysanthemum and Ginkgo biloba Leaf Exosomes

In plants, exosome secretion characteristics may vary depending on environmental factors. Since optimal conditions under which plants secrete exosomes differ depending on the plant, exosomes were purified by varying whether freeze-thaw treatment and UV treatment were performed on Ulleung chrysanthemum and Ginkgo biloba leaf, in order to establish optimal conditions through comparison of exosome yields caused by environmental stress, such as whether freeze-thaw treatment and UV treatment were performed. In order to confirm the optimal conditions, exosomes were purified in the same manner as in Example 1, except that only the corresponding conditions were varied, and the conditions are shown in Table 1 below. In Examples 4 and 7, Ulleung chrysanthemum and Ginkgo biloba leaf were purified by adding purified water, pressing, and applying an aqueous two-phase system, without freeze-thaw treatment and UV pretreatment.

**<Table 1>**

| | Raw Material | Freeze-Thaw Treatment | UV Treatment |
|---|---|---|---|
| Example 4 | Ulleung chrysanthemum | X | X |
| Example 5 | Ulleung chrysanthemum | O | X |
| Example 6 | Ulleung chrysanthemum | X | O |
| Example 7 | Ginkgo biloba leaf | X | X |
| Example 8 | Ginkgo biloba leaf | O | X |
| Example 9 | Ginkgo biloba leaf | X | O |

### Comparative Example 1: Preparation of Ulleung chrysanthemum Extract

10 g of dried Ulleung chrysanthemum was added to 100 g of purified water and extracted at 80°C for 3 hours. After extraction, reduced-pressure filtration was performed to obtain a Ulleung chrysanthemum extract, and then the extract was evaporated using a rotary evaporator to obtain a sample in powder form.

### Comparative Example 2: Preparation of Ginkgo biloba Leaf Extract

10 g of dried Ginkgo biloba leaves was added to 100 g of purified water and extracted at 80°C for 3 hours. After extraction, reduced-pressure filtration was performed to obtain a Ginkgo biloba leaf extract, and then the extract was evaporated using a rotary evaporator to obtain a sample in powder form.

### Test Example 1: Characterization of Exosomes Derived from Ulleung chrysanthemum: TEM Analysis

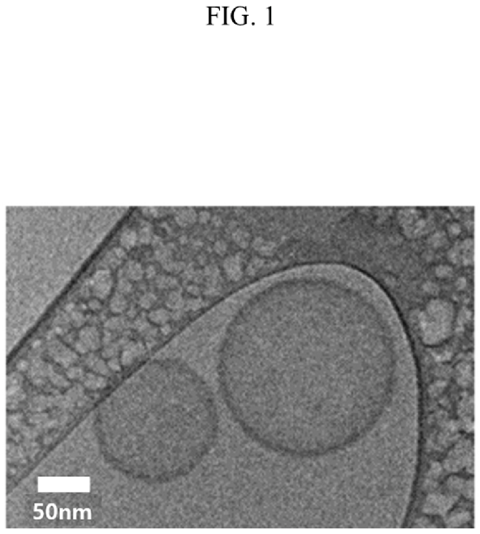

To confirm the shape of the purified exosomes derived from Ulleung chrysanthemum, the exosomes were analyzed by transmission electron microscopy (TEM). FIG. 1 is a TEM image of the exosomes derived from Ulleung chrysanthemum and purified according to Example 1. As a result of the analysis, the presence of particles having a spherical phospholipid bilayer structure and a size of about 150 nm was confirmed.

### Test Example 2: Characterization of Exosomes Derived from Ginkgo biloba Leaf: TEM Analysis

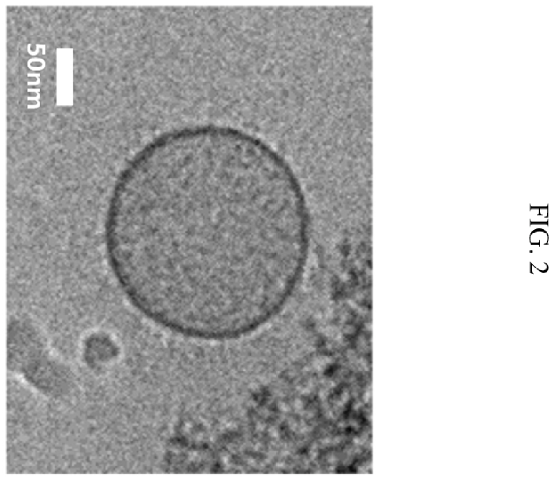

To confirm the shape of the purified exosomes derived from Ginkgo biloba leaf, the exosomes were analyzed by transmission electron microscopy (TEM). FIG. 2 is a TEM image of the exosomes derived from Ginkgo biloba leaf and purified according to Example 2. As a result of the analysis, the presence of particles having a spherical phospholipid bilayer structure and a size of about 150 nm was confirmed.

### Test Example 3: Characterization of Exosomes Derived from Ulleung chrysanthemum: NTA Analysis

The purified exosomes derived from Ulleung chrysanthemum were analyzed by nanoparticle tracking analysis (NTA) to confirm a particle size distribution and a particle number per unit volume.

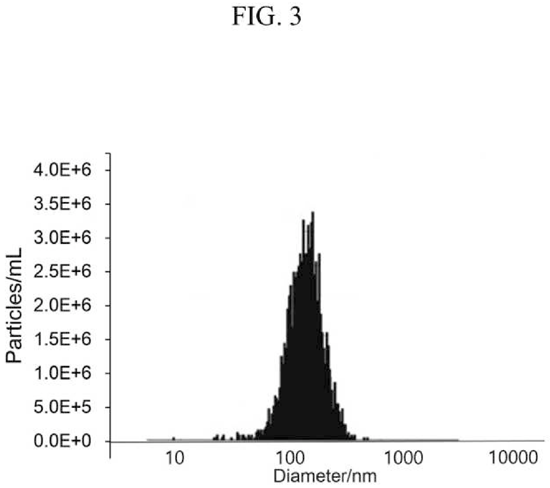

FIG. 3 is a graph showing NTA results of the exosomes derived from Ulleung chrysanthemum and purified according to Example 1. As a result of the analysis, it was confirmed that the particles had an average size of 150.1 nm and a concentration of 3.3E+10 particles per unit volume of 1 mL.

### Test Example 4: Characterization of Exosomes Derived from Ginkgo biloba Leaf: NTA Analysis

The purified exosomes derived from Ginkgo biloba leaf were analyzed by nanoparticle tracking analysis (NTA) to confirm a particle size distribution and a particle number per unit volume.

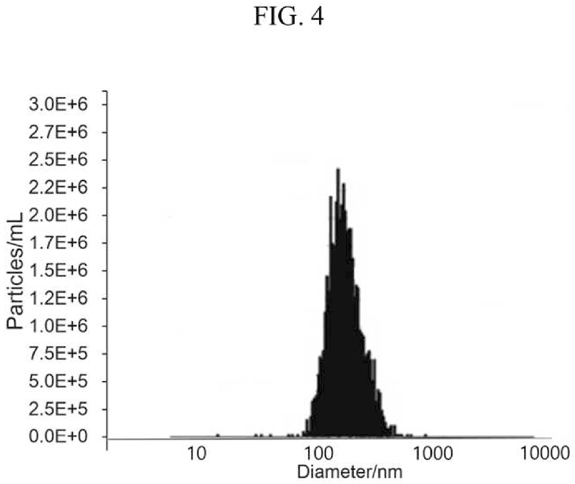

FIG. 4 is a graph showing NTA results of the exosomes derived from Ginkgo biloba leaf and purified according to Example 2. As a result of the analysis, it was confirmed that the particles had an average size of 178.5 nm and a concentration of 5.0E+10 particles per unit volume of 1 mL.

### Test Example 5: Comparison of Yields of Exosomes Derived from Ulleung chrysanthemum and Ginkgo biloba Leaf

In order to confirm optimal conditions for freeze-thaw treatment and UV treatment, the yields of exosomes derived from Ulleung chrysanthemum and Ginkgo biloba leaf and purified according to Examples 1, 2, and 4 to 9 were compared. Table 2 below shows the yield comparison as nanoparticle tracking analysis (NTA) results.

**<Table 2>**

| No. | Particle Number (Particles/ml) | Particle Size (nm) |
|---|---|---|
| Example 1 | 3.3E+10 | 150.1 |
| Example 2 | 5.0E+10 | 178.5 |
| Example 4 | 6.2E+08 | 148.7 |
| Example 5 | 3.1E+09 | 143.2 |
| Example 6 | 2.7E+09 | 147.3 |
| Example 7 | 8.0E+08 | 176.3 |
| Example 8 | 5.2E+09 | 170.6 |
| Example 9 | 6.4E+09 | 173.7 |

As confirmed in Table 2 above, the yields of exosomes were the highest in Examples 1 and 2, in which freeze-thaw treatment and UV pretreatment were performed. Based on the results of Examples 4 and 7, exosomes for which freeze-thaw treatment and UV pretreatment were not performed showed the lowest yields. This indicates that pretreatment conditions greatly affect the yield of exosomes, and it was confirmed that the optimal conditions were those in which freeze-thaw treatment and UV pretreatment were performed.

### Test Example 6: Cytotoxicity Evaluation

To confirm cytotoxicity of Ulleung chrysanthemum exosomes (Example 1), Ginkgo biloba leaf exosomes (Example 2), mixed exosomes thereof (Example 3), a Ulleung chrysanthemum extract (Comparative Example 1), and a Ginkgo biloba leaf extract (Comparative Example 2), an MTT assay was performed. Human keratinocyte (HaCaT) cells were seeded in a 96-well plate at a concentration of 1×10⁵ cells/mL and cultured at 37°C for 18 hours under 5% CO₂. After culturing, the medium was removed, each well was washed with PBS buffer, and Ulleung chrysanthemum exosomes, Ginkgo biloba leaf exosomes, mixed exosomes thereof, a Ulleung chrysanthemum extract, and a Ginkgo biloba leaf extract were added to fresh medium at different concentrations, followed by culturing again for 24 hours. To measure cell viability, MTT solution (5 mg/mL) was added, formazan formed for 4 hours was dissolved in dimethyl sulfoxide (DMSO), and absorbance was measured at 570 nm using an ELISA reader.

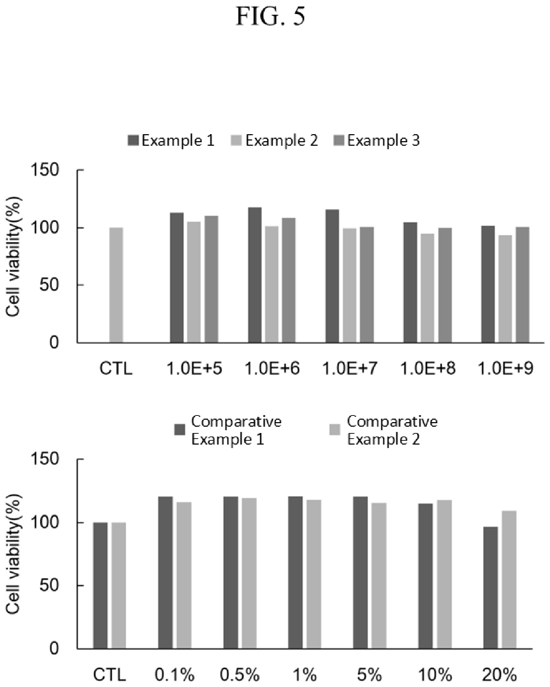

FIG. 5 is a graph showing MTT assay results for cytotoxicity of the samples. As a result of the test, by treatment with Ulleung chrysanthemum exosomes, Ginkgo biloba leaf exosomes, mixed exosomes of Ulleung chrysanthemum and Ginkgo biloba leaf, a Ulleung chrysanthemum extract, and a Ginkgo biloba leaf extract, cytotoxicity was not observed at any concentration.

### Test Example 7: Evaluation of Wrinkle-Improving Efficacy of Ulleung chrysanthemum and Ginkgo biloba Leaf Exosomes (COL1A1 Expression)

In order to confirm the wrinkle-improving efficacy of exosomes derived from Ulleung chrysanthemum and Ginkgo biloba leaf, effects on COL1A1 expression were evaluated in comparison with extracts. Human dermal fibroblast (HDFa) cells were seeded and cultured at 37°C for 24 hours under 5% CO₂ in Fibroblast Basal Medium (Medium 106) supplemented with 100 IU/mL penicillin and 100 µg/mL streptomycin. The medium in each well was removed and replaced with fresh serum-free medium. Each well was treated with Ulleung chrysanthemum exosomes, Ginkgo biloba leaf exosomes, a Ulleung chrysanthemum extract, and a Ginkgo biloba leaf extract at different concentrations, and pretreatment was performed for 4 hours. Each well plate was irradiated with 20 mJ/cm² UVB using a UVB irradiation device (Vilber Lourmat, France). After treatment with serum-free medium containing each diluted sample, the cells were further cultured for 24 hours. PBS was used as a negative control, and 10 nM TGF-β1 was used as a positive control.

After completion of cell culture, the cells were lysed using Ribo Ex^{™} Total RNA Isolation Solution (GeneAll Biotechnology, Korea) and a scraper, and then 0.2 mL of chloroform (Sigma-Aldrich, USA) was added, followed by centrifugation at 12,000 rpm and 4°C for 30 minutes. A supernatant containing RNA was separated, isopropanol (Merck-Millipore, Germany) was added in the same amount as the supernatant, and the resulting mixture was inverted and then centrifuged at 12,000 rpm and 4°C for 30 minutes. After RNA was precipitated, the supernatant was discarded while retaining the precipitate, and 70% ethanol (Merck-Millipore, Germany) was added to the remaining precipitate, followed by centrifugation at 12,000 rpm and 4°C for 10 minutes for washing. Ethanol was removed, the precipitate was dried at room temperature, and then dissolved in Nuclease-Free Water (Affymetrix, USA) to extract total RNA.

The purity and concentration of RNA were measured based on absorbance at wavelengths of 260 nm and 280 nm using a MaestroNano Micro-volume Spectrophotometer (MN-913, Maestrogen, USA), and the A260/A280 ratio was confirmed to be within the range of 2.0 to 2.2. cDNA was synthesized by preparing, in a PCR tube, 1 µg of RNA, Oligo dT (Bionics, Korea), dNTP (Takara, Korea), and nuclease-free water to a total volume of 13 µL, reacting the mixture at 65°C for 5 minutes, and then reacting the mixture at 37°C for 50 minutes using M-MLV Reverse Transcriptase (Invitrogen, Thermo Fisher Scientific, Waltham, Massachusetts, USA).

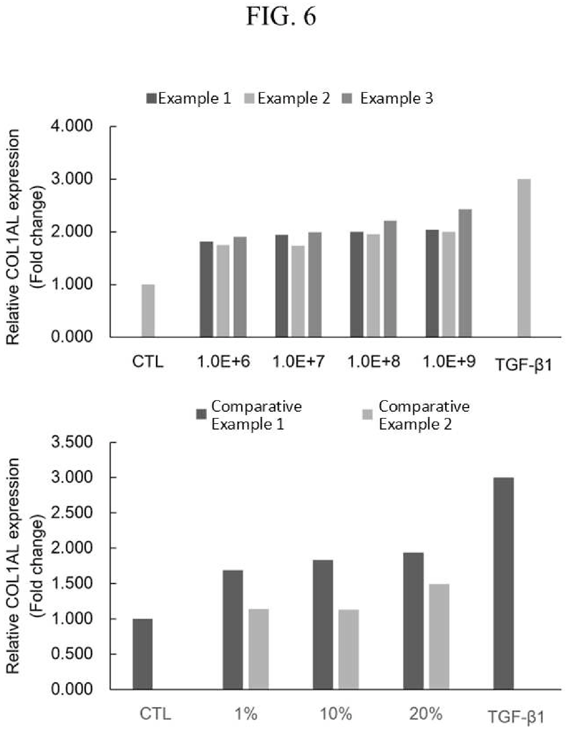

Quantitative real-time PCR (qRT-PCR) was performed to quantitatively analyze gene expression patterns in the cells treated with the samples. For qRT-PCR, a reaction solution having a total volume of 20 µL was prepared by mixing primer, cDNA, 2X SYBR Green PCR Master Mix (Applied Biosystems, USA), and HPLC (J. T. Baker, USA) in a PCR tube, and PCR was performed using a StepOnePlus Real-Time PCR System (Applied Biosystems, USA).
The PCR primer sequences for the COL1A1 gene are shown in Table 3 below, and FIG. 6 is a graph showing results of evaluating wrinkle-improving efficacy of exosomes derived from Ulleung chrysanthemum and Ginkgo biloba leaf and purified according to the above Example, based on COL1A1 mRNA expression. As a result of the test, COL1A1 mRNA, which is a collagen production factor, increased in a concentration-dependent manner by treatment with Ulleung chrysanthemum exosomes, Ginkgo biloba leaf exosomes, a Ulleung chrysanthemum extract, and a Ginkgo biloba leaf extract, and it was confirmed that the expression rate was much higher in the exosome treatment concentration range than in the extract treatment. In the positive control treated with 10 nM TGF-β1, the fold change value was confirmed to be 2.998.

**<Table 3>**

| Gene | Forward primer (5'→3') | Reverse primer (5'→3') |
|---|---|---|
| COL1A1 | 5'-GAG AGC ATG ACC GAT GGA TT- 3' | 5'-CCT TCT TGA GGT TGC CAG TC-3' |
| GAPDH | 5'-GTC  TCC  TCT  GAC  TTC  AAC AGC G-3' | 5'-ACC ACC CTG TTG CTG TAG CCA A-3' |

### Test Example 8: Evaluation of Wrinkle-Improving Efficacy of Ulleung chrysanthemum and Ginkgo biloba Leaf Exosomes (Inhibition of MMP-1 Expression)

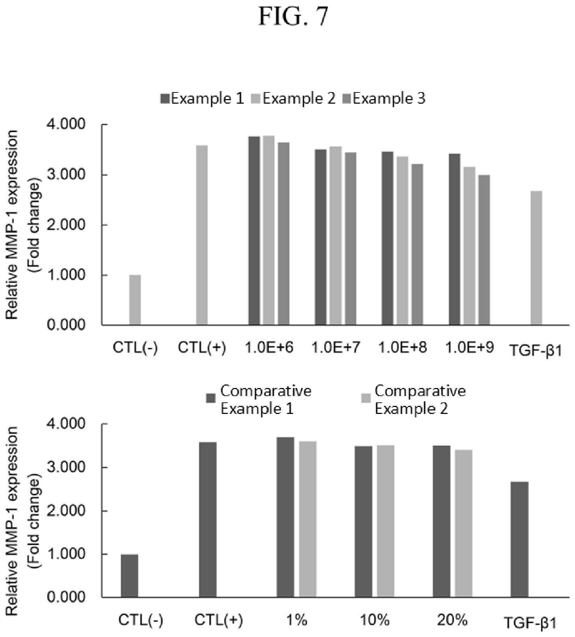

In order to confirm the wrinkle-improving efficacy of exosomes derived from Ulleung chrysanthemum and Ginkgo biloba leaf, effects on MMP-1 expression were confirmed in comparison with extracts. The experimental method was the same as in Test Example 7, and the PCR primer sequences for the MMP-1 gene are shown in Table 4 below. FIG. 7 is a graph showing results of evaluating wrinkle-improving efficacy of exosomes derived from Ulleung chrysanthemum and Ginkgo biloba leaf and purified according to the above Examples, based on inhibition of MMP-1 mRNA expression. As a result of the test, MMP-1 mRNA, which is a collagenase factor, decreased in a concentration-dependent manner by treatment with Ulleung chrysanthemum exosomes, Ginkgo biloba leaf exosomes, a Ulleung chrysanthemum extract, and a Ginkgo biloba leaf extract, and it was confirmed that the inhibition rate was much higher in the exosome treatment concentration range than in the extract treatment. In the positive control treated with 10 nM TGF-β1, the fold change value was confirmed to be 2.680.

**<Table 4>**

| Gene | Forward primer (5'→3') | Reverse primer (5'→3') |
|---|---|---|
| MMP-1 | 5'-GGG CTT GAA GCT GCT TAC GA-3' | 5'-ACA GCC CAG TAC TTA TTC CCT TTG-3' |
| GAPDH | 5'-GTC TCC TCT GAC TTC AAC AGC G-3' | 5'-ACC ACC CTG TTG CTG TAG CCA A-3' |

### Test Example 9: Evaluation of Skin Barrier-Enhancing Efficacy of Ulleung chrysanthemum and Ginkgo biloba Leaf Exosomes (Filaggrin Expression)

In order to confirm the skin barrier-enhancing efficacy of exosomes derived from Ulleung chrysanthemum and Ginkgo biloba leaf, effects on Filaggrin expression were evaluated in comparison with extracts. Human keratinocyte (HaCaT) cells were seeded and cultured at 37°C for 24 hours under 5% CO₂ in Fibroblast Basal Medium (Medium 106) supplemented with 100 IU/mL penicillin and 100 µg/mL streptomycin. The medium in each well was removed and replaced with fresh serum-free medium. Each well was treated with Ulleung chrysanthemum exosomes, Ginkgo biloba leaf exosomes, a Ulleung chrysanthemum extract, and a Ginkgo biloba leaf extract at different concentrations, and pretreatment was performed for 4 hours. Each well plate was irradiated with 20 mJ/cm² UVB using a UVB irradiation device (Vilber Lourmat, France). After treatment with serum-free medium containing each diluted sample, the cells were further cultured for 24 hours. PBS was used as a negative control, and 100 nM CaCl₂ was used as a positive control.

After completion of cell culture, the cells were lysed using Ribo Ex^{™} Total RNA Isolation Solution (GeneAll Biotechnology, Korea) and a scraper, and then 0.2 mL of chloroform (Sigma-Aldrich, USA) was added, followed by centrifugation at 12,000 rpm and 4°C for 30 minutes. A supernatant containing RNA was separated, isopropanol (Merck-Millipore, Germany) was added in the same amount as the supernatant, and the resulting mixture was inverted and then centrifuged at 12,000 rpm and 4°C for 30 minutes. RNA was precipitated, the supernatant excluding the precipitate was discarded, and 70% ethanol (Merck-Millipore, Germany) was added to the remaining precipitate, followed by centrifugation at 12,000 rpm and 4°C for 10 minutes for washing. Ethanol was removed, the precipitate was dried at room temperature, and then dissolved in Nuclease-Free Water (Affymetrix, USA) to extract total RNA.

The purity and concentration of RNA were measured based on absorbance at wavelengths of 260 nm and 280 nm using a MaestroNano Micro-volume Spectrophotometer (MN-913, Maestrogen, USA), and the A260/A280 ratio was confirmed to be within the range of 2.0 to 2.2. cDNA was synthesized by preparing, in a PCR tube, 1 µg of RNA, Oligo dT (Bionics, Korea), dNTP (Takara, Korea), and nuclease-free water to a total volume of 13 µL, reacting the mixture at 65°C for 5 minutes, and then reacting the mixture at 37°C for 50 minutes using M-MLV Reverse Transcriptase (Invitrogen, Thermo Fisher Scientific, Waltham, Massachusetts, USA).

Quantitative real-time PCR (qRT-PCR) was performed to quantitatively analyze gene expression patterns in the cells treated with the samples. For qRT-PCR, a reaction solution having a total volume of 20 µL was prepared by mixing primer, cDNA, 2X SYBR Green PCR Master Mix (Applied Biosystems, USA), and HPLC (J. T. Baker, USA) in a PCR tube, and PCR was performed using a StepOnePlus Real-Time PCR System (Applied Biosystems, USA).

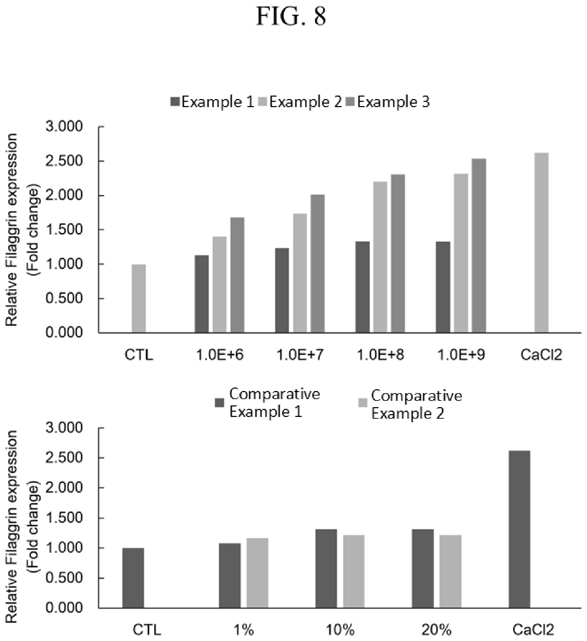

The PCR primer sequences for the Filaggrin gene are shown in Table 5 below, and FIG. 8 is a graph showing results of evaluating skin barrier-enhancing efficacy of exosomes derived from Ulleung chrysanthemum and Ginkgo biloba leaf and purified according to the above Examples, based on Filaggrin mRNA expression. As a result of the test, Filaggrin mRNA, which is a skin barrier protein, increased in a concentration-dependent manner by treatment with Ulleung chrysanthemum exosomes, Ginkgo biloba leaf exosomes, a Ulleung chrysanthemum extract, and a Ginkgo biloba leaf extract, and it was confirmed that expression was much higher in the exosome treatment concentration range than in the extract treatment. In the positive control treated with 100 nM CaCl₂, the fold change value was confirmed to be 2.620.

**<Table 5>**

| Gene | Forward primer (5'→3') | Reverse primer (5'→3') |
|---|---|---|
| Filaggrin | 5'-GCT GAA GGA ACT TCT GGA AAA GG-3' | 5'-GTT GTG GTC TAT ATC CAA GTG ATC-3' |
| GAPDH | 5'-GTC TCC TCT GAC TTC AAC AGC G-3' | 5'-ACC ACC CTG TTG CTG TAG CCA A-3' |

### Test Example 10: Evaluation of Skin Barrier-Enhancing Efficacy of Ulleung chrysanthemum and Ginkgo biloba Leaf Exosomes (Loricrin Expression)

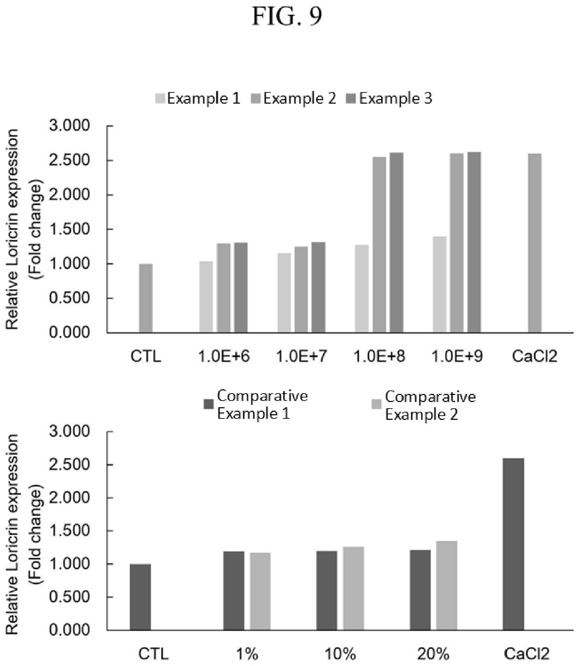

In order to confirm the skin barrier-enhancing efficacy of exosomes derived from Ulleung chrysanthemum and Ginkgo biloba leaf, effects on Loricrin expression were confirmed in comparison with extracts. The experimental method was the same as in Test Example 9, and the PCR primer sequences for the Loricrin gene are shown in Table 6 below. FIG. 9 is a graph showing results of evaluating skin barrier-enhancing efficacy of exosomes derived from Ulleung chrysanthemum and Ginkgo biloba leaf and purified according to the above Examples, based on Loricrin mRNA expression. As a result of the test, Loricrin mRNA, which is a skin barrier protein, increased in a concentration-dependent manner by treatment with Ulleung chrysanthemum exosomes, Ginkgo biloba leaf exosomes, a Ulleung chrysanthemum extract, and a Ginkgo biloba leaf extract, and it was confirmed that expression was much higher in the exosome treatment concentration range than in the extract treatment. In the positive control treated with 100 nM CaCl₂, the fold change value was confirmed to be 2.600.

**<Table 6>**

| Gene | Forward primer (5'→3') | Reverse primer (5'→3') |
|---|---|---|
| Loricrin | 5'- GTC TGC GGA GGT GGT TCC TCT-3' | 5'- TGC TGG GTC TGG TGG CAG ATC-3' |
| GAPDH | 5'-GTC TCC TCT GAC TTC AAC AGC G-3' | 5'-ACC ACC CTG TTG CTG TAG CCA A-3' |

### Test Example 11: Evaluation of Cell-Regenerating Efficacy of Ulleung chrysanthemum and Ginkgo biloba Leaf Exosomes (Wound Healing Assay)

In order to confirm the cell-regenerating efficacy of exosomes derived from Ulleung chrysanthemum and Ginkgo biloba leaf, a wound healing assay was performed. When cells are damaged by chemical and physical stimuli, the cells secrete various chemokines and cytokines and, at the same time, migrate to a skin-damaged site to induce cell proliferation. Accordingly, a cell regeneration-inducing effect of the samples was confirmed through a wound healing assay for testing the growth and migration of skin cells.

Human keratinocyte (HaCaT) cells were seeded in a 6-well plate and cultured at 37°C for 24 hours under 5% CO₂. After culturing, the cells were scratched using a 200 µL pipette tip. After scratching, detached cells were removed by repeated washing with PBS buffer, and the medium was replaced with DMEM medium containing 1.5% bovine serum, followed by treatment with Ulleung chrysanthemum exosomes, Ginkgo biloba leaf exosomes, a Ulleung chrysanthemum extract, and a Ginkgo biloba leaf extract. As a positive control, treatment was performed with 10 ng/mL TGF-β1.

Thereafter, the cells were cultured for 18 hours, the same position was photographed with a microscope at 0 hours and 18 hours after a wound was formed, and the reduction in wound area over 18 hours was measured using Image J (National Institutes of Health, USA) to calculate an average value.

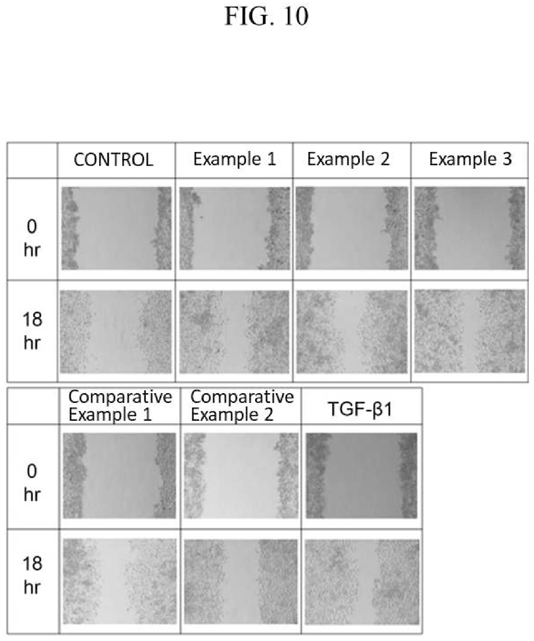

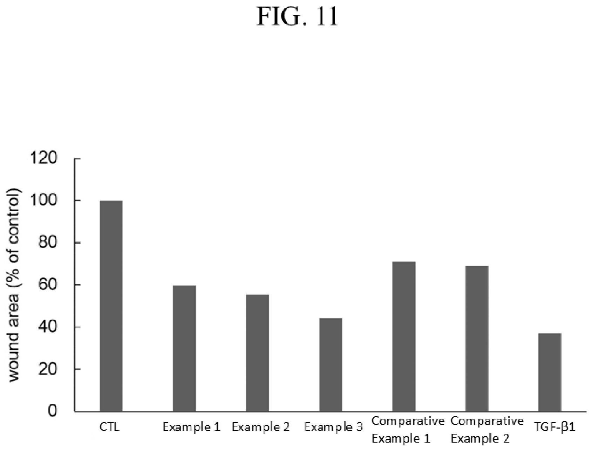

FIG. 10 is an image showing wound healing assay results for skin-regenerating efficacy of exosomes derived from Ulleung chrysanthemum and Ginkgo biloba leaf and purified according to the above Examples, and shows results obtained by treatment with Ulleung chrysanthemum exosomes and Ginkgo biloba leaf exosomes at 1.0E+08 particles/mL and treatment with Ulleung chrysanthemum extract and Ginkgo biloba leaf extract at 20%. FIG. 11 is a graph showing results for a wound area ratio.

As a result of the test, the wound area was decreased by treatment with exosomes derived from Ulleung chrysanthemum and exosomes derived from Ginkgo biloba leaf, and a higher area reduction rate was shown by treatment with mixed exosomes of Ulleung chrysanthemum and Ginkgo biloba leaf. Accordingly, it was confirmed that the growth and migration of cells were increased, thereby having an effect on cell regeneration. In the positive control treated with 10 ng/mL TGF-β1, an area ratio of 37.05% was shown.

### Formulation Examples 1 to 3: Preparation of Creams

Creams containing Ulleung chrysanthemum exosomes and Ginkgo biloba leaf exosomes purified according to the above Examples were prepared by a conventional method with the compositions shown in Table 7 below. A cream containing the Ulleung chrysanthemum extract of Comparative Example 1 was used as Comparative Formulation Example 1, and a cream containing the Ginkgo biloba leaf extract of Comparative Example 2 was used as Comparative Formulation Example 2.

**<Table 7>**

| Ingredient | Formulation Example 1 | Formulation Example 2 | Formulation Example 3 | Comparative Formulation Example 1 | Comparative Formulation Example 2 |
|---|---|---|---|---|---|
| | Content (% by weight) | | | | |
| Ulleung chrysanthemum Exosomes (Example 1) | 5 | - | - | - | - |
| Ginkgo biloba Leaf Exosomes (Example 2) | - | 5 | - | - | - |
| Mixed Exosomes of Ulleung chrysanthemum and Ginkgo biloba Leaf (Example 3) | - | - | 5 | - | - |
| Ulleung chrysanthemum Extract (Comparative Example 1) | - | - | - | 5 | - |
| Ginkgo biloba Leaf Extract (Comparative Example 2) | - | - | - | - | 5 |
| Glycerin | 10 | 10 | 10 | 10 | 10 |
| Butylene Glycol | 5 | 5 | 5 | 5 | 5 |
| Glyceryl Oleate | 1.8 | 1.8 | 1.8 | 1.8 | 1.8 |
| Cetearyl Olivate | 0.5 | 0.5 | 0.5 | 0.5 | 0.5 |
| Sorbitan Olivate | 0.5 | 0.5 | 0.5 | 0.5 | 0.5 |
| Caprylic/Capric Triglyceride | 5.0 | 5.0 | 5.0 | 5.0 | 5.0 |
| Cetyl Ethylhexanoate | 1.0 | 1.0 | 1.0 | 1.0 | 1.0 |
| Beeswax | 0.5 | 0.5 | 0.5 | 0.5 | 0.5 |
| Squalane | 0.2 | 0.2 | 0.2 | 0.2 | 0.2 |
| 1,2-Hexanediol | 0.2 | 0.2 | 0.2 | 0.2 | 0.2 |
| Cholesteryl/Behenyl /Octyldodecyl Lauroyl Glutamate | 1.0 | 1.0 | 1.0 | 1.0 | 1.0 |
| Dimethicone | 0.5 | 0.5 | 0.5 | 0.5 | 0.5 |
| Cyclopentasiloxane/ Cyclohexasiloxane | 2.0 | 2.0 | 2.0 | 2.0 | 2.0 |
| Cetearyl Alcohol | 1.0 | 1.0 | 1.0 | 1.0 | 1.0 |
| Mineral Oil | 2.5 | 2.5 | 2.5 | 2.5 | 2.5 |
| Disodium EDTA | 0.02 | 0.02 | 0.02 | 0.02 | 0.02 |
| BHT | 0.05 | 0.05 | 0.05 | 0.05 | 0.05 |
| Tocopheryl Acetate | 0.3 | 0.3 | 0.3 | 0.3 | 0.3 |
| Panthenol | 0.2 | 0.2 | 0.2 | 0.2 | 0.2 |
| Ethylhexyl Methoxycinnamate | 0.2 | 0.2 | 0.2 | 0.2 | 0.2 |
| Fragrance | 0.01 | 0.01 | 0.01 | 0.01 | 0.01 |
| Purified Water | Balance | Balance | Balance | Balance | Balance |

### Test Example 12: Evaluation of Wrinkle Improvement of Exosomes Derived from Ulleung chrysanthemum and Ginkgo biloba Leaf

In order to confirm the wrinkle-improving efficacy of Ulleung chrysanthemum exosomes (Example 1), Ginkgo biloba leaf exosomes (Example 2), and mixed exosomes thereof (Example 3), the creams prepared in the above Formulation Examples were applied to both sides of the faces of 20 adult women in their 30s to 50s, and red light having a wavelength of 633 nm was irradiated using an LED light source device for a cumulative time of 24 hours over 6 weeks. Thereafter, wrinkle improvement effects were evaluated through an actual use test.

Table 8 shows results of evaluating wrinkle improvement effects of exosomes derived from Ulleung chrysanthemum and Ginkgo biloba leaf and purified according to the above Examples through an actual use test.

**<Table 8>**

| | Wrinkle Improvement Effect | | |
|---|---|---|---|
| | Excellent | Slight | None |
| Formulation Example 1 | 6 | 10 | 4 |
| Formulation Example 2 | 8 | 8 | 4 |
| Formulation Example 3 | 12 | 7 | 1 |
| Comparative Formulation Example 1 | 2 | 4 | 14 |
| Comparative Formulation Example 2 | 3 | 4 | 13 |

As can be seen from the results in Table 8 above, creams containing the Ulleung chrysanthemum exosomes, the Ginkgo biloba leaf exosomes, and the mixed exosomes of Ulleung chrysanthemum and Ginkgo biloba leaf of the present invention exhibited excellent skin wrinkle improvement effects.

## Claims

1. A cosmetic composition comprising, as an active ingredient, Ulleung chrysanthemum exosomes, Ginkgo biloba leaf exosomes, or a mixture thereof.

2. The cosmetic composition of claim 1, wherein the Ulleung chrysanthemum exosomes and the Ginkgo biloba leaf exosomes as the active ingredient are purified by a method comprising:
(A) adding a solvent to Ulleung chrysanthemum or Ginkgo biloba leaves and extracting by repeating freezing and thawing;
(B) treating Ulleung chrysanthemum or Ginkgo biloba leaves subjected to freeze-thaw pretreatment with UV-A for 1 hour to 6 hours;
(C) pressing the UV-treated Ulleung chrysanthemum or Ginkgo biloba leaves to obtain a pressed juice;
(D) centrifuging the pressed juice at 1,000×g to 10,000×g to obtain a supernatant;
(E) lyophilizing the supernatant in which exosomes are present;
(F) forming an aqueous two-phase system using polyethylene glycol (PEG)/dextran in the lyophilized product; and
(G) obtaining a lower phase in which exosomes are concentrated from the aqueous two-phase system.

3. The cosmetic composition of claim 2, wherein the freeze-thaw treatment in step (A) is performed by repeating, 2 to 5 times, a process of freezing at a temperature of -80°C to -70°C for 15 hours to 20 hours and then thawing at a temperature of 40°C to 50°C for 8 hours to 10 hours.

4. The cosmetic composition of claim 2, wherein the UV treatment in step (B) is performed with UV-A having a wavelength of 340 nm to 380 nm for 4 hours to 6 hours.

5. The cosmetic composition of claim 1, wherein the Ulleung chrysanthemum exosomes, the Ginkgo biloba leaf exosomes, or the mixture thereof are contained in an amount of 0.0001% to 30.0% (w/w) based on a total weight of the composition.

6. The cosmetic composition of claim 1, wherein the cosmetic composition is for wrinkle improvement.

7. The cosmetic composition of claim 1, wherein the cosmetic composition is for cell regeneration.

8. The cosmetic composition of claim 1, wherein the cosmetic composition is for skin barrier enhancement.

9. A method for purifying Ulleung chrysanthemum or Ginkgo biloba leaf exosomes, the method comprising:
(A) adding a solvent to Ulleung chrysanthemum or Ginkgo biloba leaves and extracting by repeating freezing and thawing;
(B) treating Ulleung chrysanthemum or Ginkgo biloba leaves subjected to freeze-thaw pretreatment with UV-A for 1 hour to 6 hours;
(C) pressing the UV-treated Ulleung chrysanthemum or Ginkgo biloba leaves to obtain a pressed juice;
(D) centrifuging the pressed juice at 1,000×g to 10,000×g to obtain a supernatant;
(E) lyophilizing the supernatant in which exosomes are present;
(F) forming an aqueous two-phase system using polyethylene glycol (PEG)/dextran in the lyophilized product; and
(G) obtaining a lower phase in which exosomes are concentrated from the aqueous two-phase system.

10. The method of claim 9,
wherein the freeze-thaw treatment in step (A) is performed by repeating, 2 to 5 times, a process of freezing at a temperature of -80°C to -70°C for 15 hours to 20 hours and then thawing at a temperature of 40°C to 50°C for 8 hours to 10 hours, and
wherein the UV treatment in step (B) is performed with UV-A having a wavelength of 340 nm to 380 nm for 4 hours to 6 hours.
